# EUROPÄISCHE PATENTSCHRIFT

(11) **EP 2 593 827 B1**
(45) Veröffentlichungstag und Bekanntmachung des Hinweises auf die Patenterteilung: **31.08.2016**
(21) Anmeldenummer: 11730911.2
(22) Anmeldetag: 07.07.2011
(51) Int. Cl.: A61B 1/00, G02B 13/00, G02B 5/00, G02B 5/04, G02B 23/24

(54) **OBJEKTIV EINES SCHRÄG BLICKENDEN, STARREN ENDOSKOPES**
OBJECTIVE OF AN ANGULARLY VIEWING, RIGID ENDOSCOPE
OBJECTIF D'UN ENDOSCOPE RIGIDE À VISÉE OBLIQUE

(30) Priorität: 13.07.2010 DE 102010027079
(43) Veröffentlichungstag der Anmeldung: 22.05.2013
(73) Patentinhaber: Olympus Winter & Ibe GmbH, 22045 Hamburg (DE)
(72) Erfinder: SCHOUWINK, Peter, 22307 Hamburg (DE); ZHAO, Jianxin, 22041 Hamburg (DE)
(74) Vertreter: Hausfeld, Norbert
(86) Internationale Anmeldenummer: PCT/EP2011/003373
(87) Internationale Veröffentlichungsnummer: WO 2012/007126

(56) Entgegenhaltungen:
- EP-A1- 0 571 725
- DE-A1- 3 640 186
- DE-A1- 19 720 163
- JP-A- 2 108 013

## Beschreibung

Die Erfindung betrifft ein Objektiv der im Oberbegriff des Anspruches 1 genannten Art.

Objektive von Endoskopen weisen eine Reihe von Problemen auf, wie z.B., dass Endoskope meist sehr dünn sein sollen, was dazu rührt, dass auch die Objektivlinsen im Durchmesserbereich weniger Millimeter liegen müssen. Bei starren Objektiven kommt das Problem hinzu, dass diese zumeist schrägblickend ausgebildet sein müssen, wobei die Blickrichtung des Objektives in einem Winkel zur Achse des Objektivs steht, in der Strahlen durch den Hauptteil des Objektivs nach proximal abgegeben werden, z. B. unmittelbar auf einen Bildempfänger oder auf einen das Bild über die Länge des Endoskopes führenden Bildleiter. Die Abwinkelung der Strahlen zwischen diesen beiden Richtungen erfolgt üblicherweise mit einer Prismenanordnung.

Die DE 197 20 163 A1, Fig. 3, zeigt ein gattungsgemäßes Objektiv, bei dem die Prismenanordnung aus zwei Prismen besteht, von denen das proximale Prisma die Strahlen nach zweimaliger Reflexion an Spiegeln in Richtung der Achse des Objektivs abgibt. Die DE 35 37 155 C2 zeigt eine ähnliche Konstruktion mit einer Absorptionsschicht.

Die JP 2 108013 A zeigt in Fig. 13 eine gattungsgemäße Konstruktion, bei der an der Kontaktfläche zwischen den beiden Prismen eine Blende angeordnet ist, die den Durchgangsbereich begrenzt.

An dem zweiten Spiegel treten bisher ungelöste Probleme auf. Der zweite Spiegel muss in Richtung auf den Durchgangsbereich der Strahlen durch die Kontaktflächen begrenzt sein, da er sonst die Strahlen teilweise unterbrechen würde. Ragt der zweite Spiegel jedoch nicht weit genug in die Richtung des Durchgangsbereiches, dann können schräg zur Blickrichtung einfallende Strahlen am zweiten Spiegel vorbei und auch am ersten Spiegel vorbei unmittelbar durch das proximale Prisma hindurch in proximale Richtung zum Betrachter hin laufen und dort störende Effekte erzeugen. Aus diesem Grund sollte also der zweite Spiegel möglichst weit an den Durchgangsbereich heranreichen. Allerdings stört dann ein anderer Effekt. Schräge Strahlen, die sehr steil durch die Kontaktflächen verlaufen, können im proximalen Prisma zwischen den Spiegeln so steil verlaufen, dass sie noch einmal zusätzlich hin und her laufen, also insgesamt viermal reflektiert werden. Dies führt ebenfalls zu Störungen. Das Problem der Vierfachreflexion wird um so stärker, je kleiner der Schrägblickwinkel ist. Der Konstrukteur befindet sich hier also in einem Dilemma zwischen zwei störenden Effekten, die entweder bei zu langem oder bei zu kurzem Spiegel auftreten. Eine mittlere Länge ist aber ebenfalls unbefriedigend, da dann beide Effekte auftreten können.

Die Aufgabe der vorliegenden Erfindung besteht darin, bei einem gattungsgemäßen Objektiv die sich aus der Länge des zweiten Spiegels ergebenden Probleme zu vermeiden.

Diese Aufgabe wird mit den Merkmalen des Kennzeichnungsteiles des Anspruches 1 gelöst.

Erfindungsgemäß ist in dem Bereich neben dem Durchgangsbereich, in dem der zweite Spiegel fehlt und somit ein Strahl direkt an beiden Spiegeln vorbei durchlaufen könnte, eine Absorptionsschicht vorgesehen, die einen gerade durchlaufenden Strahl absorbiert. Die Absorptionsschicht verhindert aber auch die Vierfachreflexion im proximalen Prisma, da bei besonders steilen Strahlen diese nach der ersten Reflexion auf die Absorptionsschicht treffen und dort absorbiert werden. Auf diese konstruktiv sehr einfache Weise werden also beide störenden Effekte gleichzeitig bekämpft.

Die Absorptionsschicht könnte angrenzend an den zweiten Spiegel angeordnet sein, ist vorzugsweise aber gemäß Anspruch 2 diesen überlappend angeordnet. Da der Spiegel zum proximalen Prisma hin reflektieren muss, kann sie nur auf der dem distalen Prisma zugewandten Außenseite des Spiegels angeordnet sein. Dort stört die Absorptionsschicht nicht, sondern wirkt sogar vorteilhaft dadurch, dass sie innere Reflexionen an diesem Spiegel im distalen Prisma verhindert.

Der Spiegel und die überlappende Absorptionsschicht könnten beide übereinander auf der Kontaktfläche des distalen Prismas oder der des proximalen Prismas angeordnet sein. Vorteilhaft gemäß Anspruch 3 ist jedoch der Spiegel auf dem proximalen Prisma und die Absorptionsschicht auf dem distalen Prisma angeordnet, was eine einfache Herstellung erlaubt.

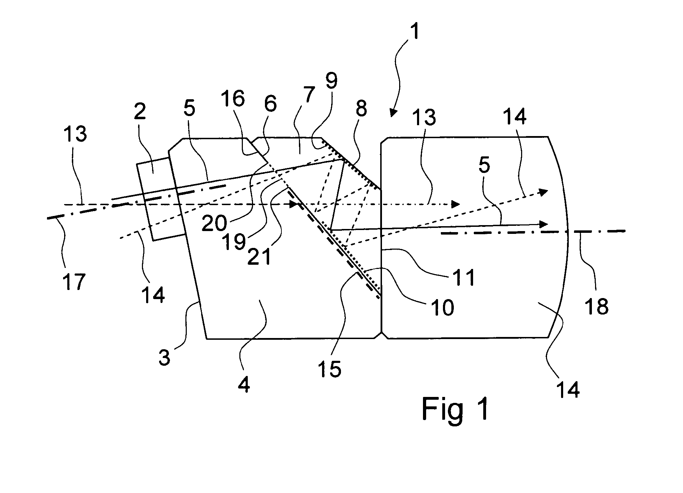

In der Zeichnung ist die Erfindung beispielsweise und schematisch in einer einzigen Fig. 1 dargestellt, die einen Achsschnitt durch den distalen Teil eines erfindungsgemäßen Objektivs zeigt.

Fig. 1 zeigt den distalen Endbereich eines Objektivs 1 eines starren Endoskopes.

Es wird auf die Figuren 1 bis 3 der DE 197 20 163 A1 Bezug genommen. Im Vergleich mit den Figuren 2 und 3 dieser Schrift erkennt man, dass das in der Fig. 1 dieser Anmeldung gezeigte Objektivteil der distale Endbereich der bekannten Konstruktion ist.

In dem stark schematisiert dargestellten Bereich des Objektivs 1 tritt das Licht durch eine Negativlinsenanordnung 2 ein, die auf der distalen Stirnfläche 3 eines distalen Prismas 4 befestigt ist. Ein mittlerer Bildstrahl 5 verläuft parallel zur Blickrichtung 17 des Objektivs 1 durch die Negativlinsenanordnung 2, durch das distale Prisma 4 und in ein dahinter liegendes proximales Prisma 7. Dabei durchsetzt der Bildstrahl 5 eine Kontaktfläche 6 des Prismas 4 und eine Kontaktfläche 16 des Prismas 7. Die Prismen 4, 7 liegen mit diesen planen Kontaktflächen 6, 16 aneinander.

Der Strahl 5 trifft dann auf eine Schrägfläche 8 des proximalen Prismas 7, die mit einem nach innen reflektierenden ersten Spiegel 9 versehen ist, der als gepunktete Linie dargestellt ist. Nach dortiger Reflexion fällt der Strahl 5 auf einen zweiten Spiegel 10, der ebenfalls als gepunktete Linie dargestellt ist und der auf der Kontaktfläche des proximalen Prismas 7 angeordnet ist. Von dort wird der Strahl 5 weiter in proximaler Richtung zum Betrachter reflektiert. Im dargestellten Beispiel liegt der Strahl 5 nun in der Achse 18 des Objektivs und fällt durch eine Querfläche 11, die senkrecht zur Achse 18 des Objektivs steht, in eine Linse 12, von der er weiter in den nicht dargestellten proximalen Teil des Objektivs verläuft, der in Fig. 3 der genannten Schrift mit dem Bezugszeichen 35 bezeichnet ist.

Wie Fig. 1 zeigt, steht die distale Stirnfläche 3 des distalen Prismas 4 senkrecht zur Blickrichtung 17 des Objektivs, die schräg zu seiner Achse 18 steht. Es handelt sich hier um ein Objektiv mit sehr kleinem Schrägblickwinkel.

Insoweit wie bisher beschrieben, stimmt die in Fig. 1 dargestellt Konstruktion mit der der Fig. 3 der zitierten Schrift überein. Dabei können zwei störende Probleme auftreten. Ein schräger Strahl 13, der parallel zur Achse des Objektivs eintritt, kann am oberen Ende des zweiten Spiegels 10 und am unteren Ende des ersten Spiegels 9 vorbei geradeaus durch das proximale Prisma 7 bis zum Betrachter verlaufen. Das ergibt starke Störungen.

Würde man den Spiegel 10 in Richtung zum Durchgangsbereich der Strahlen durch die Kontaktflächen 6, 16 verlängern, dann würde ein zweiter störender Effekt auftreten.

Ein weiterer schräger Strahl 14, der in anderer Richtung derart liegt, dass er steiler auf den ersten Spiegel 9 auftritt, wird mit engem Reflexionswinkel so reflektiert, dass er nicht wie der reguläre Strahl 5 zweimal reflektiert wird, also einmal am ersten Spiegel 9 und einmal am zweiten Spiegel 10, sondern einmal mehr hin und her läuft, so dass er insgesamt viermal reflektiert wird, wie anhand des Strahles 14 dargestellt.

Ist der Spiegel 10 so kurz, wie in Fig. 1 dargestellt, kann, wie Fig. 1 zeigt, diese Vierfachreflexion nicht passieren. Wird er aber verlängert, um das Hindurchtreten des Strahles 13 zu verhindern, dann kommt es zur Vierfachreflexion des Strahles 14.

Um den Strahl 13 zu verhindern, müsste der Spiegel 10 also verlängert werden. Um die Vierfachreflexion des Strahles 14 zu verhindern, müsste er wieder verkürzt werden. Es besteht ein konstruktives Dilemma.

In Fig. 1 ist gestrichelt ein Flächenbereich der Kontaktflächen 6, 16 als Durchgangsbereich 19 markiert. Der Durchgangsbereich 19 erstreckt sich in der Darstellung der Fig. 1 zwischen den Punkten 20 und 21. Der Durchgangsbereich 19 wird von ordnungsgemäss das Objektiv durchlaufenden Strahlen durchsetzt.

Der Flächenbereich der Kontaktflächen 6, 16, der jenseits des Punktes 21 an den Durchgangsbereich 19 angrenzt, ist kritisch sowohl für ohne Reflexion gerade durchgehende Strahlen, wie den Strahl 13, als auch für vierfach reflektierte Strahlen wie den Strahl 14. Erfindungsgemäss ist dort eine gestrichelt dargestellte Absorptionsschicht 15 vorgesehen. Diese absorbiert, wie in Fig. 1 zu sehen ist, den Strahl 13, aber auch den störend eng reflektierten Strahl 14. Die Absorptionsschicht 15 kann ohne Weiteres bis eng an den Durchgangsbereich 19 heranreichen und somit sowohl den direkt durchgehenden Strahl 13 als auch den vierfach reflektierten Strahl 14 in breitem Winkelbereich blockieren.

Die Absorptionsschicht 15 darf nicht in den Durchgangsbereich 19 reichen, da sie dort nutzbare Strahlen absorbieren würde, sie sollte auf jeden Fall aber den kritischen Flächenbereich zwischen dem Durchgangsbereich 19 und dem zweiten Spiegel 10 überdecken. Wie dargestellt, kann die Absorptionsschicht 15 aber auch darüber hinaus den Spiegel 10 überlappend angeordnet sein, also parallel zu diesem denselben Flächenbereich der Kontaktflächen 6, 16 bedecken.

Der Spiegel 9 ist als Metallbeschichtung auf der Außenseite der Schrägfläche 8 des proximalen Prismas 7 angeordnet. Ebenso ist der zweite Spiegel 10 von außen auf dessen Kontaktfläche 16 aufgebracht. Die Absorptionsschicht 15 ist auf der Kontaktfläche 6 des distalen Prismas 4 angeordnet und besteht z.B. aus Schwarzchrom.

## Patentansprüche

1. Objektiv (1) eines schräg blickenden, starren Endoskopes, mit einer Prismenanordnung bestehend aus zwei mit planen, schräg zur Achse (18) des Objektivs (1) stehenden Kontaktflächen (6, 16) aneinanderliegenden Prismen (4, 7), wobei das distale Prisma (4) eine senkrecht zur Blickrichtung (17) des Objektivs (1) stehende distale Stirnfläche (3)aufweist, von der dort eintretende Strahlen (5) durch das distale Prisma (4) und durch einen Durchgangsbereich (19) der Kontaktflächen (6, 16) in das proximale Prisma (7) eintreten und nach zweimaliger Reflexion in diesem an einem ersten Spiegel (9) an einer Schrägfläche (8) und einem zweiten Spiegel (10) an der Kontaktfläche (16) in Richtung der Achse (18) des Objektivs (1) durch eine senkrecht zu dieser stehende Fläche (11) austreten, wobei der zweite Spiegel (10) außerhalb des Durchgangsbereiches 19 angeordnet ist, **dadurch gekennzeichnet, dass** in der Ebene (6, 16) des zweiten Spiegels (10) in einem zwischen dem Spiegel (10) und dem Durchgangsbereich (19) liegenden Flächenbereich eine Absorptionsschicht (15) angeordnet ist.

2. Objektiv nach Anspruch 1, **dadurch gekennzeichnet, dass** die Absorptionsschicht (15) den zweiten Spiegel (10) überlappend angeordnet ist.

3. Objektiv nach Anspruch 2, **dadurch gekennzeichnet, dass** der zweite Spiegel (10) als Beschichtung auf der Kontaktfläche (16) des proximalen Prismas (7) und die Absorptionsschicht (15) auf der Kontaktfläche (6) des distalen Prismas (4) angeordnet ist.

## Claims

1. Objective (1) of an angularly viewing, rigid endoscope, comprising a prism arrangement consisting of two adjoining prisms (4, 7), which lie against each other at flat contact surfaces (6, 16) that are at an angle to the axis (18) of the objective (1), wherein the distal prism (4) has a distal end face (3), which is perpendicular to the viewing direction (17) of the objective (1), from which distal end face beams (5) incident thereon enter the proximal prism (7) through the distal prism (4) and through a passage area (19) of the contact surfaces (6, 16)
and after being reflected twice in said the proximal prism at a first mirror (9) on an angled surface (8) and at a second mirror (10) on the contact surface (16), exit through a surface (11) perpendicular to the axis of the objective in the direction of the axis (18) of the objective (1),
the second mirror (10) is arranged outside of the passage area (19),
**characterised in that**
- an absorption layer (15) is arranged in the plane (6, 16) of the second mirror (10) in a surface area located between the mirror (10) and the passage area (19).

2. Objective according to claim 1, **characterised in that** the absorption layer (15) is arranged so as to overlap the second mirror (10).

3. Objective according to claim 2, **characterised in that** the second mirror (10) is arranged as coating on the contact surface (16) of the proximal prism (7) and the absorption layer (15) is arranged on the contact surface (6) of the distal prism (4).

## Revendications

1. Objectif (1) d'un endoscope rigide à visée oblique avec un agencement de prismes composé de deux prismes contigus (4, 7) avec des surfaces de contact (6, 16) planes obliques par rapport à l'axe (18) de l'objectif (1), le prisme distal (4) présentant une face frontale distale (3) perpendiculaire à la direction de visée (17) de l'objectif (1), les rayons (5) qui entrent par cette face frontale traversant le prisme distal (4) et entrant par une zone de passage (19) des surfaces de contact (6, 16) dans le prisme proximal (7) puis, après une double réflexion dans celui-ci par un premier miroir (9) sur une surface oblique (8) et par un second miroir (10) sur la surface de contact (16), ressortant en direction de l'axe (18) de l'objectif (1) par une surface (11) perpendiculaire à cet axe, le second miroir (10) étant placé en dehors de la zone de passage 19, **caractérisé en ce qu'**une couche d'absorption (15) est agencée dans le plan (6, 16) du second miroir (10) dans une zone de surface située entre le miroir (10) et la zone de passage (19).

2. Objectif selon la revendication 1, **caractérisé en ce que** la couche d'absorption (15) est agencée de façon à chevaucher le second miroir (10).

3. Objectif selon la revendication 2, **caractérisé en ce que** le second miroir (10) est formé par un revêtement sur la surface de contact (16) du prisme proximal (7) et la couche d'absorption (15) est placée sur la surface de contact (6) du prisme distal (4).
